# EUROPEAN PATENT SPECIFICATION

(11) **EP 4 043 638 B1**
(45) Date of publication and mention of the grant of the patent: **05.11.2025**
(21) Application number: 21216785.2
(22) Date of filing: 22.12.2021
(51) Int. Cl.: B32B 5/02, B32B 7/05, D06M 15/643, D06N 3/00, D06N 3/12, A41D 13/12, A61B 46/00, B32B 5/26

(54) **COATED BARRIER FABRIC FOR A REUSABLE MEDICAL PRODUCT**
BESCHICHTETES BARRIEREGEWEBE FÜR EIN WIEDERVERWENDBARES MEDIZINISCHES PRODUKT
TISSU BARRIÈRE ENDUIT POUR UN PRODUIT MÉDICAL RÉUTILISABLE

(30) Priority: 28.12.2020 US 202017135069
(43) Date of publication of application: 17.08.2022
(73) Proprietor: Standard Textile Co., Inc., Cincinnati, OH 45237 (US)
(72) Inventor: Stewart, Richard, Mason (US); Bushman, Bradley J., Cincinnati (US)
(74) Representative: Wallinger Ricker Schlotter Tostmann

(56) References cited:
- EP-A1- 0 507 760
- EP-A2- 0 330 783
- EP-A2- 1 192 958
- WO-A1-2006/028706
- WO-A2-2006/078280
- WO-A2-2013/071266
- US-A- 4 991 232
- US-A- 5 236 532
- US-A1- 2005 278 837
- US-A1- 2006 041 989
- US-B1- 6 374 828

## Description

### Technical Field

This application relates generally to barrier fabrics and, more specifically, to coated barrier fabrics for use in reusable medical products.

### Background

Barrier fabrics are generically characterized as being resistant to penetration by liquids. Because of this property, barrier fabrics are especially suited for use in the medical field to prevent or control the spread of infectious microorganisms, such as viruses and bacteria found in blood, and other potentially infectious material ("OPIM") associated with, for example, surgical procedures.

Barrier fabric properties are critical for medical products such as surgical drapes that are used to maintain sterile surgical or procedure fields and protective apparel such as surgical or isolation gowns. Particularly where there is a possibility of coming into contact with bodily fluids, every effort is made to protect the health professional and the patient. Health professionals routinely use medical barrier fabrics during surgery, the drawing of blood, or while working with specimens containing contaminated fluids to both protect themselves and to avoid cross or secondary contamination of subsequent patients through the inadvertent transmission of infectious materials.

Generally, there are two types of medical barrier fabrics: (1) single use, i.e., disposable, fabrics, and (2) reusable fabrics. Performance of the single use or disposable fabrics in terms of liquid resistance is generally acceptable; however, these fabrics often fail to provide the spectrum of properties deemed necessary to achieve desirable protection in many medical applications. Additionally, single use items contribute significantly to the volume of medical waste and are inconsistent with accepted principles of environmental sustainability. Reusable medical barrier fabrics, on the other hand, can offer equivalent or better performance with respect to liquid resistance, better fabric drape, wearer comfort, and lower cost per use. However, it is also known that reusable barrier fabrics are commonly associated with diminished protective performance over the course of the usable service life.

Importantly, reusable medical gowns, surgical drapes, and other non-disposable medical barrier fabric products have requirements that distinguish them from other products or garments that incorporate barrier fabrics. To wit, after each use, a reusable surgical gown, for example, must be washed, dried, and sterilized for subsequent reuse. These procedures often involve harsh detergents and high temperatures which can quickly degrade the barrier properties of the gown and limit the number of times the gown can be reused.

A typical, institutional laundering/autoclaving cycle for such reusable medical products generally comprise one or more initial flushes in which the products are soaked in water at 32 to 38 °C (90°-100° F) for two to five minutes. Following the one or more flushes, the products are soaked in an alkali bath at approximately 49 to 71 °C (120°-160° F) for three to ten minutes to loosen dirt. Next the products are placed in a detergent bath at approximately 71 °C(160° F) for approximately five to ten minutes. This is followed by one or more rinsings at temperatures that may be progressively reduced from about 60 °C (140° F) to ambient temperature. The products are mechanically agitated in some, if not all of these baths. Also, following each bath, there is a drain to minimize the liquid carried over to the succeeding process. Finally, there is an acid sour bath in which the pH is adjusted to the 4.0 to 7.0 range, and in which a softening agent may also be employed. After washing, the products are extracted (spin or hydraulic press) to remove as much water as possible prior to drying. The products are then dried in a tumbling dryer at an average temperature of 71 °C (160° F). Typical drying times for products are in the order of 15 to 30 minutes. It is to be noted that there can be hot spots in such dryers, which can subject the products to temperatures in excess of 204 °C (400° F). After drying, the products are placed in an autoclave and sterilized by pressurized steam at a temperature of approximately 132 °C (270° F) for at least four minutes in a commercially available pre-vacuum steam sterilizer. Parameters for sterilization will differ for a gravity steam sterilizer.

These harsh conditions are several orders of magnitude greater than those that exist in the laundering or dry cleaning of barrier fabrics incorporated in ordinary garments. In fact, many of the barrier fabrics intended for use in normal (e.g., non-medical) garments, such as foul weather gear, become unusable after a single, or relatively few, institutional laundering/autoclaving cycles.

While the efforts to produce reusable barrier fabrics with required liquid and microorganism resistance for use in medical applications have seen some success, fabric coatings known in the art tend to degrade the feel of the fabric and give the coated fabric a rubberized finish, which is not appealing for many fabric uses, particularly garments. Moreover, heavy abrasion or multiple harsh institutional laundering/autoclave cycles often result in breakdowns in the protective properties of some known reusable barrier fabrics, which can significantly shorten the potential useful life of the fabrics.

It thus would be beneficial to provide an improved coated barrier fabric, such as for use in a reusable medical product, that achieves excellent liquid resistance without sacrificing the comfort or the feel of the fabric. Further, the fabric should be able to sustain said barrier properties after at least 10 institutional laundering/autoclaving cycles without sacrificing the comfort or the feel of the fabric.

WO 2006/078280 A2 relates to a porous adaptive membrane structure that has moveable membranes.

WO 2006/028706 A1 relates to a protective-apparel sleeve including a tubular inner piece of membrane laminated or coated fabric and a tubular outer piece of membrane laminated or coated fabric generally surrounding the tubular inner piece.

US 2006/041989 A1 relates to a liquid-proof protective-apparel sleeve including a tubular inner piece of membrane laminated or coated fabric and a tubular outer piece of membrane laminated or coated fabric generally surrounding the tubular inner piece.

US 5 236 532 A relates to a liquid impermeable barrier fabric which resists penetration of liquids under finite hydraulic heads and maintains that capable after upwards of 75 institutional washing/sterilization cycles.

### Summary

The present invention relates generally to barrier fabrics and, more specifically, to coated barrier fabrics for use in reusable medical products.

In the invention according to claim 1 a coated barrier fabric is provided that includes a first ply and a second ply each comprising a coated side and a non-coated side, wherein the first ply and the second ply are joined together about a periphery of the first ply and the second ply to form the barrier fabric and the coated side of the first ply and the coated side of the second ply comprise a non-fluorine containing polymer coating of silicone, wherein the coated side of the first ply and the coated side of the second ply face each other and define an interior of the barrier fabric, with each coated side able to come into direct contact with one another but remain movable thereagainst in a transverse direction and able to form a gap therebetween, wherein the non-coated side of the first ply and the non-coated side of the second ply face opposing directions and define an exterior of the barrier fabric, wherein the non-fluorine containing polymer coating is applied at a rate from 15 g/m2 to 50 g/ m2, and wherein the barrier fabric acts as a barrier against bodily fluids.

In the invention according to claim 9 a reusable medical product is provided that includes the barrier fabric of at least one of claims 1 to 8.

In the invention according to claim 14 a method for making a two-ply barrier fabric for use in a reusable medical product is provided and includes applying, via a single pass, a non-fluorine containing polymer coating of silicone to a side of each of two separate plies of a woven or knitted fabric, wherein the coating is applied at a rate of from 15 g/m² to 50 g/m². Next, the coating on the fabric is cured, by a single cure process and then the coated first ply and second ply are joining together about a periphery of each of the first ply and the second ply to form the barrier fabric, wherein the coated side of the first ply and the coated side of the second ply face each other and define an interior of the barrier fabric, with each coated side able to come into direct contact with one another but remain movable thereagainst in a transverse direction and able to form a gap therebetween. wherein the non-coated side of the first ply and the non-coated side of the second ply face opposing directions and define an exterior of the barrier fabric, and wherein the barrier fabric acts as a barrier against bodily fluids and wherein the barrier fabric is suitable for use within or as a reusable medical product. The polymer coating is silicon and applied at a rate of from 15 g/m² to 50 g/m². In another example, the content of the first and second ply comprise at least 25% polyester or at least 25% polyamide.

### Brief Description of the Drawings

The accompanying drawings, which are incorporated in and constitute a part of this specification, illustrate embodiments of the invention and, together with a Detailed Description of the embodiments given below, serve to explain the principles of the invention.
FIG. 1 is a front elevational view of a surgical gown, which includes a coated barrier fabric, in accordance with an embodiment of the present invention;
FIG. 2 is an enlarged, fragmentary top plan view of the incircle portion 2 of the gown of FIG. 1 showing a coated barrier woven fabric;
FIG. 3A is a cross-sectional view of a portion of the gown of FIG. 1, showing a gap between the plies of the coated fabric of the gown; and
FIG. 3B is a cross-sectional view of a portion of the gown of FIG. 1 similar to FIG. 3A, but showing no gap between the plies of the coated fabric of the gown.

### Detailed Description

The exemplary embodiments described herein are provided for illustrative purposes and are not limiting. Other exemplary embodiments are possible, and modifications may be made to the exemplary embodiments within the scope of the present disclosure. Therefore, this Detailed Description is not meant to limit the scope of the present disclosure.

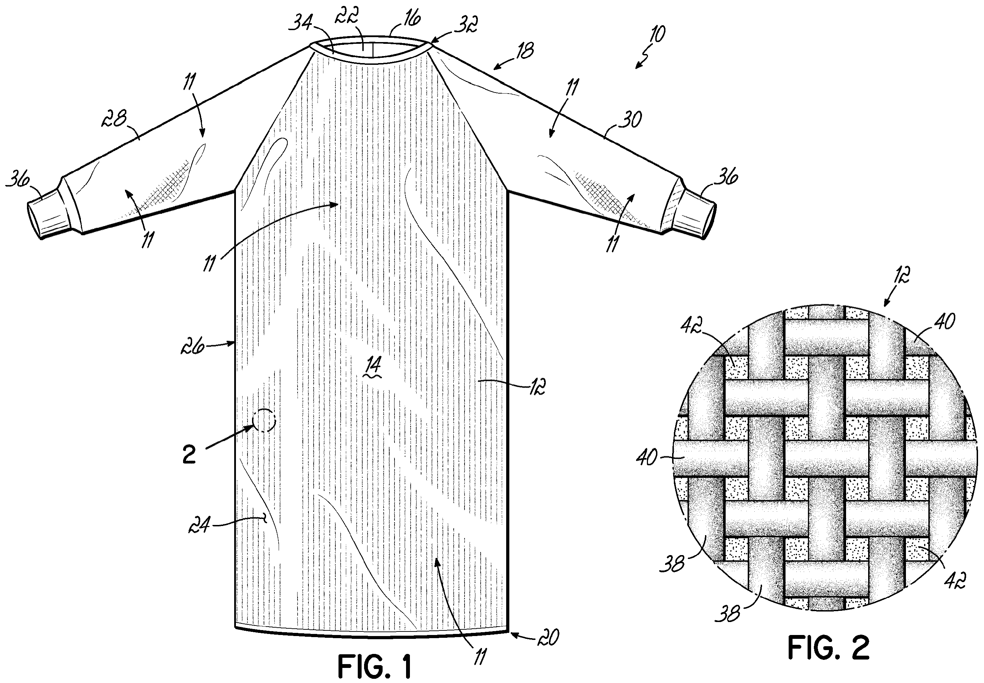

FIGS. 1 through 3B show a reusable surgical gown 10, or portions thereof, in accordance with an embodiment of the present invention. At least some portions of the gown 10, are fabricated from a coated, two-ply barrier fabric 12. The fabric 12 offers a reliably durable and effective liquid barrier without sacrificing comfort or feel (as known coated or laminated barrier fabrics in the art do) due to the fabric's 12 coated two-ply construction. Moreover, the fabric 12 exhibits the durability to withstand repeated institutional laundering/autoclave cycles while maintaining effective liquid barrier properties. Other advantages and technical effects of the embodiments of this invention will become evident to one skilled in the art from the following description.

Use of descriptive terms hereinbelow such a left, right, top, bottom, front, back, or vertical as the terms pertain to or describe the gown 10 are to be interpreted from the viewpoint of a wearer of the gown 10, when properly donned, unless otherwise noted.

With specific reference to FIG. 1, the reusable surgical gown 10 generally includes a front 14 and a back 16, a top 18 and a bottom 20, and an inner surface 22 and an outer surface 24. The gown 10 also includes a central body 26 and a pair of opposing long sleeves, i.e., right sleeve 28 and left sleeve 30. Each of the sleeves 28, 30 are located adjacent to and extend in a direction away from a neck opening 32. The neck opening 32 is generally defined by the top edge 34 of the central body 26. Further, the sleeves 28, 30 each have an optional terminal cuff 36. The sleeves 28, 30 may be joined to the central body 26 and the cuffs 36 may be joined to their respective sleeves 28, 30 by techniques known in the art, such as by sewing.

One or more of the terminal cuffs 36, in other embodiments, may be absent altogether, with the long sleeves 28, 30 simply defining an opening that is hemmed at a distal end of each sleeve 28, 30. In another example, the long sleeves 28, 30 may be short sleeves or they may be no sleeves at all, if so desired. In other embodiments, the terminal cuff 36 may be replaced with long sleeves 28, 30 having thumb loops, for example. Moreover, although the central body 26 and the sleeves 28, 30 are shown as one piece in FIG. 1, it should be appreciated that the central body 26 or sleeves 28, 30 could be formed of multiple fabric pieces stitched or fastened together, or the like, by means known in the art.

Still referring to FIG. 1, at least some portions of the gown 10, such as certain critical zones 11, can be fabricated from the coated, two-ply barrier fabric 12. The construction and composition of the fabric 12 is further described below in relation to FIGS. 2-3B. As used herein, a critical zone 11 is an area of a medical product, such as a gown or drape, where the product is most likely to come into direct contact with potentially infectious material. According to the Association for the Advancement of Medical Instrumentation ("AAMI") PB70 Standard, the critical zone 11 of a surgical gown, such as the gown 10 depicted in FIG. 1, are, at minimum, the front of the gown from chest to knees and the sleeves of the gown from the cuff to above the elbow.

It will be appreciated that alternative embodiments can take forms other than a reusable surgical gown 10. For example, the fabric 12 may be alternatively embodied in other reusable medical products such as a surgical drape, stand or table covers, wrappers, or other protective apparel. In the case of an isolation gown, the AAMI PB70 Standard dictates that the critical zones 11 are the entire gown, including the seams, but excluding the cuffs, hems, and bindings. In the case of a surgical drape, the critical zone 11 can be defined as the area of the drape generally surrounding the fenestration. Regardless of the embodiment, it is to be understood that the critical zones 11 of embodiments (*e.g.,* gowns, drapes, etc.) may be fabricated from a coated, two-ply barrier fabric 12. However, the fabric 12 may be used in other embodiments (besides in the critical zones 11 of gowns and drapes). In this way, the fabric 12 is not limited in use to any specific reusable medical product.

Referring now to FIG. 2, the figure shows an enlarged view of a portion of the reusable surgical gown 10 of FIG. 1. Specifically, FIG. 2 shows one ply 44, *i.e.,* a top or outer ply, of the fabric 12 that is used to fabricate at least the critical zones 11 of the gown 10. In the embodiment depicted in FIG. 2, the fabric 12 is defined by a woven web of warp yarns 38 and fill (or weft) yarns 40. In an embodiment, at least some of the yarns are multifilament yarns, though other yarns (e.g., monofilament) may be used. In an alternative embodiment, the fabric 12 may be knitted, or the like, as opposed to woven. It is to be understood that other fabric constructions known in the art may be used. Regardless of how the fabric 12 is produced (*e.g.,* woven, knitted, etc.), in an embodiment, the content of the yarn that makes up the fabric 12 is 25% or more filament polyester or polyamide. In one example, the yarn of one ply 44, 46 may include polyester and the yarn of the other ply 44, 46 may include polyamide, or, in another example, the yarns of any one ply 44, 46 may include combinations of polyester and polyamide. In an alternative embodiment, the content of the yarn that makes up the fabric 12 is up to 100% filament polyester or polyamide. Polyester or polyamide, such as nylon, may be chosen due to their material properties (*e.g.,* lint generation performance, colorfastness, inherent hydrophobicity, etc.), wide availability, and cost. It is to be understood that other non-linting polymers or fibers could be used.

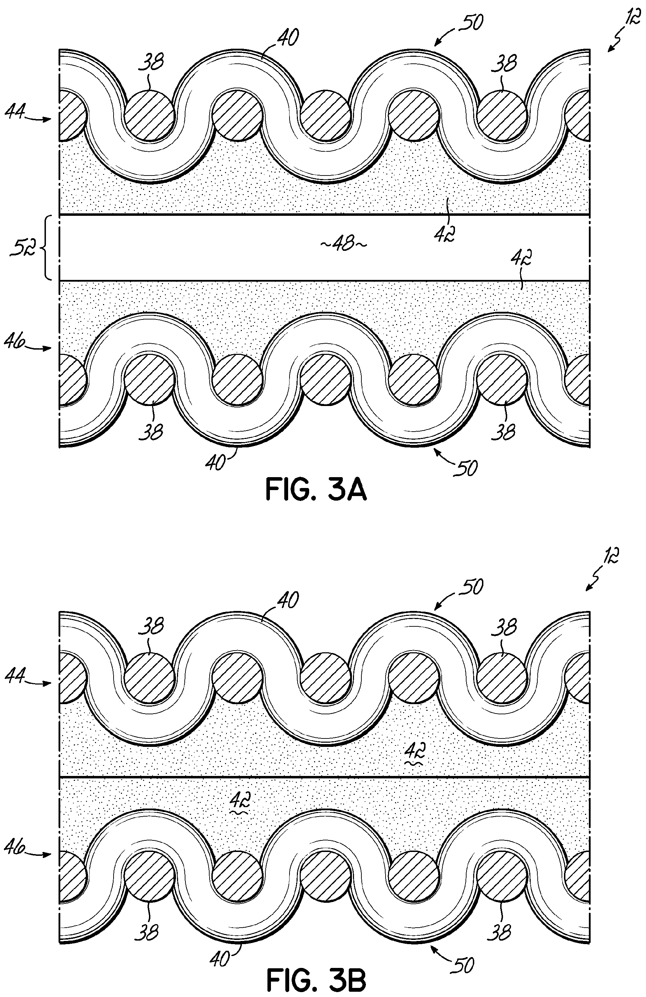

Referring now to FIGS. 3A and 3B, the figures show a cross-sectional portion in a critical zone 11 of an embodiment of the gown 10. More specifically, the figures show the coated, two-ply barrier fabric 12 from which the selected portion of the gown 10 is constructed. The fabric 12 is comprised of two plies 44 and 46, *i.e.,* a first ply 44 of woven material and a second ply 46 of woven material with each ply having a coating 42 thereon. Together, the two plies 44, 46 define an interior 48 of the fabric 12 and an exterior 50 of the fabric 12. The interior 48 of the fabric 12 is the region between the first ply 44 and the second ply 46 that is generally shielded from the surrounding environment by virtue of the two-ply construction of the fabric 12. The exterior 50 of the fabric 12 is the region on opposing sides of the first and second plies 44, 46 that is exposed to the surrounding environment.

As can be seen in FIGS. 3A and 3B, only one side of each of the first and second plies 44, 46 is coated with a coating 42. In alternative embodiments, only one of the two plies 44, 46 may feature a coating 42 on one side of a ply. However, in this embodiment, one side of the first ply 44 is coated with a coating 42 and one side of the second ply 46 is coated with a coating 42. The coated side of the first ply 44 and the coated side of the second ply 46 face each other in the interior 48 of the fabric 12. In this construction, the coating 42 on the first and second plies 44, 46 is not exposed to the environment. Instead, the coating 42 on the first and second plies 44, 46 is interiorly protected. Such a construction offers substantial advantages over other barrier fabrics known in the art. This coating-to-coating orientation diminishes abrasion to the coating material and extends product service life. Specifically, the useful life of the gown 10 (or other garment, drape, etc. the fabric 12 is used in) can be extended because the coating 42 is not exposed to outside or external elements, such as during the laundering/autoclave cycle, that typically lead to degradation of the coating 42 (and thus the loss of the "barrier" properties of the fabric). Further, the comfort and feel of the gown 10 (or other garment, drape, etc.) embodying the fabric 12 are improved as each or either ply of the product may have a coating weight that is lighter than has historically been the case. Additionally, a wearer of the gown 10, for example, is exposed to the non-coated (*e.g.,* woven, knitted, etc.) sides of the fabric 12-thereby eliminating direct contact with the coating 42 materials by the wearer or user of a finished product (as in the case of some known reusable barrier fabrics). In one example, the wearer of the gown 10 is exposed only to a non-coated side(s) of the fabric 12. Generally, wearers disfavor the feeling of the coating 42 against their skin, especially for long periods of time (as can be the case with gowns 10 and other surgical or medical apparel in medical or surgical applications). As such, the fabric 12 as embodied in the gown 10 offers improved comfort and feel over other barrier fabrics known in the art.

The material for the coating 42 can be applied to one side of each of the first and second plies 44, 46 via conventional coating technologies to impart a solid, semisolid, liquid, or vaporized (*e.g.,* sublimation) chemistry to coat one side of the fabric 12, as is known in the industry. For example, the coating 42 may be applied to the fabric 12 using a knife coat, spray coat, foam coat, or similar methodology. It will be understood that alternative methodologies, as generally known in the art, besides those specifically listed can be used to apply the coating 42 to one side of each of the first and second plies 44, 46 of the fabric 12. Upon application, the material for the coating 42 can interact with the surface of the ply 44, 46 in any number of ways, as would be understood in the industry, to provide the coating 42. For example, the applied coating material can adhere to the surface of the ply 44, 46, and may form a layer thereover, such as by binding to surface fibers (*e.g., via* covalent and/or non-covalent bonds, etc.) or the coating material can become entrapped within the fibers of the ply 44, 46. Further, a combination of some of all of polymerization, bonding, and physical entrapment can be used to adhere the coating 42 to the plies 44, 46.

Further, when applying the coating 42, it is desirable to limit the penetration of the coating 42 through the ply 44, 46. It is known in the industry that applying a coating to a side of one ply 44, 46 can result in an undesirable "bleed-through" effect in which the applied coating may penetrate therethrough and appear on the other side of the ply 44, 46. It will be understood that the term 'coated' as used herein excludes the bleed-through of the coating 42. It will also be understood that the term 'non-coated' is inclusive of the bleed-through of coating 42 material. For example, if a coating 42 is applied to one side of a ply 44, 46, then the side of the ply 44, 46 that the coating 42 was applied to would be considered 'coated'; however, the opposing side of the ply 44, 46, to which a coating 42 was not applied, could be considered 'non-coated', even if it exhibits bleed through effects from the coating 42 on the opposing side of the ply 44, 46.

Still referring to FIGS. 3A and 3B, the coating 42 can be applied to the fabric in a single pass and dried or cured thereon. In one example, the coating 42 can be applied in a single pass and single cure application. Here, the coating 42 may be heated, as is known in the art, to desirably cure the applied coating 42 on each ply 44, 46, where cure time and temperature, for example, may be generally dependent upon the selected coating material. A single pass and single cure application of the coating 42 offers distinct advantages over a two (or more) pass and two (or more) cure application, as may be used with barrier fabrics known in the art. Specifically, a single pass and cure operation desirably provides cost and efficiency benefits over an application that requires additional passes and time for curing. Instead of two (or more passes), the coating 42 can be applied to one side of the first or second ply 44, 46 in a single pass. This reduces production time and thereby reduces the cost of producing the coated fabric 12. Similarly, the coated fabric 12 can be cured in a single curing process as opposed to two (or more) cures-at least one for each application pass. This also reduces production time and thereby reduces the cost of producing the coated fabric 12. Such benefits translate to the ability to produce more coated fabrics 12 in a shorter amount of time without sacrificing the quality of the coating 42. Thus, the overall cost and the per use cost of the fabric 12 are reduced, making reusable medical products embodying the fabric 12 attractive alternatives to disposable barrier fabrics or other reusable barrier fabrics known in the art.

When the coating 42 is applied to a ply (first or second ply 44, 46 of the fabric 12), the coating 42 is applied at a rate of from 15 g/m² to 50 g/m². In another embodiment, the coating 42 can be applied at a rate of from 15 g/m² to 35 g/m². In a further embodiment, the coating 42 can be applied at a rate of from 20 g/m² to 30 g/m². Even more specifically, in an embodiment, the coating 42 can be applied at a rate of 15, 20, or 25 g/m². Further, in an embodiment, the total, cumulative weight of the coating 42 across both the first ply 44 and second ply 46, combined, can be 100 g/m² or less. In another embodiment, the total, cumulative weight of the coating 42 across both the first ply 44 and second ply 46, combined, can be 75 g/m² or less. In yet another embodiment, the total, cumulative weight of the coating 42 across both the first ply 44 and second ply 46, combined, can be 50 g/m² or less. For example, the coating 42 on the first and second plies 44, 46 may be 20 g/m² each-for a total of 40 g/m² across both plies 44, 46, and performed in a single pass. Alternatively, the coating rate on each ply 44, 46 may vary. For example, the coating 42 on the first ply 44 may be 20 g/m² and the coating 42 on the second ply 46 may be 25 g/m²-for a total of 45 g/m² across both plies 44, 46, and performed in a single pass.

Applying such a light coating 42 to the first and second plies 44, 46 of the fabric 12 results in plies 44, 46 with a desirable comfort and feel. Some known coated fabrics used in reusable medical products feature thick coatings. As such, these fabrics have an unpleasant rubber-like feel. Specifically, medical apparel made from such thickly coated fabrics are uncomfortable to wear, especially for long durations of time (such as during a medical procedure). In contrast, applying a light coating 42 on the first and second plies 44, 46 (as opposed to a thicker coating on a single ply) results in a finished fabric 12 that is similar in feel and comfort to an uncoated fabric, but with the desirable barrier properties of a coated fabric 12. As such, the fabric 12 achieves the desirable properties of a reusable barrier fabric without sacrificing on comfort or feel.

Still referring to FIGS. 3A and 3B, the coating 42 is a fluorine free coating. Other known coated barrier fabrics often utilize chemical hydrophobic finishes containing fluorocarbons, such coatings are known to diminish in effectiveness through repeated processing and use cycles. To avoid such known issues with fluorine containing coatings, the material for the coating 42, is silicone. The coating is silicone.

Referring now specifically to FIG. 3A, the figure shows the coated, two-ply barrier fabric 12 from which the selected portion of the gown 10 is constructed with a gap 52 between the first ply 44 and the second ply 46 of the fabric 12. The gap 52 is not necessarily to scale in the figure. The presence of the gap 52 in the interior 48 of the fabric 12 signifies that the first ply 44 and second ply 46 are not adhered or bonded to each other in this embodiment. The first ply 44 and the second ply 46 still are joined about their peripheries by techniques known in the art, such as by sewing. However, the plies 44, 46 are not otherwise constrained with respect to each other. Specifically, the first ply 44 and the second ply 46 are free to move against each other in the interior 48 of the fabric 12. Accordingly, there are no additional plies or layers situated between the first and second plies 44, 46.

Because only the interior-facing sides of the first and second plies 44, 46 are coated, it is understood that the coating 42 of the first ply 44 will rub, but still move freely due to coating characteristics, against the coating 42 of the second ply 46. Such is preferable to the coated side of either ply being directly exposed to the outside or exterior environment. Coatings 42 are generally abraded and/or degraded when they come into contact with any number of environmental factors. For example, if the coated side of the ply were on the exterior 50 of the fabric 12 as opposed to the interior 48, then the coating 42 would be exposed during the institutional laundering/autoclave cycle where the coating 42 could be damaged by the harsh detergents, agitation during a washing phase, or heat during a drying phase. Such abuse could severely diminish the useful life of the fabric 12 as a barrier fabric by degrading the coating 42.

Still referring to FIG. 3A, the coated sides of each of the plies 44, 46 face the interior of the fabric 12, and each other. It is understood that the coating 42 is generally not abrasive or generally substantially detrimental to itself. As such, when the coated side of the first ply 44 and the coated side of the second ply 46 rubs or moves against each other in the interior 48 of the fabric 12, the contact should be generally non-abrasive or non-degrading, i.e., generally non-detrimental. That is to say that, in general, the coated sides of the plies 44, 46 are free to interact with each other without worry of detrimentally damaging the coatings 42 during the useful laundering life of the gown 10. In this way, the construction of the fabric 12 (e.g., with the coating 42 only on the interior-facing sides of the plies 44, 46) lends itself to exceptional durability, at least in comparison to some known reusable barrier fabrics, and does not detract from the useful life of the gown 10 or whatever product, apparel, or garment the fabric 12 is embodied in (*e.g.,* a gown 10 or drape). Such is an advantage of the fabric 12 over reusable barrier fabrics known in the art.

Referring now specifically to FIG. 3B, the figure shows a cross-section of a portion of an embodiment of the gown 10, and more specifically and in contrast to FIG. 3A, the figure shows the coated, two-ply barrier fabric 12 from which the selected portion of the gown 10 is constructed with no gap 52 between the first ply 44 and the second ply 46 of the fabric 12. Despite the absence of a physical gap 52 between the first ply 44 and the second ply 46, the plies 44, 46 are not adhered or bonded together. But rather, the plies 44, 46 are free to move against each other (*e.g.,* in the transverse direction).

Referring generally to FIGS. 1-3B, at least some portions of the gown 10 are fabricated from a coated, two-ply barrier fabric 12. The two-ply construction of the fabric 12 offers advantages of other reusable barrier fabrics known in the art. Specifically, the fabric 12 offers redundancy in the form of two plies 44, 46 that each comprise a coated side. The redundancy of having each of the two plies 44, 46 coated on one side provides for a more reliable fabric 12 with a longer useful life than other coated barrier fabrics known in the art. Further to that end, it is a feature of the fabric 12 to exhibit excellent barrier fabric properties. The two-ply configuration of the fabric 12 results in a higher hydrostatic resistance than either of the two plies 44, 46 would yield individually or would be yielded by a single ply that was coated with a thicker coating 42. In one embodiment, a finished garment (*e.g.,* gown 10) or drape fabricated from the fabric 12 will test at not less than 20 cm of hydrostatic resistance within a critical zone 11 of the respective gown 10 or drape when tested pursuant to the American Association of Textile Chemists and Colorists ("AATCC") 127 hydrostatic pressure standardized test method. Similarly, in another embodiment, a finished garment (*e.g.,* gown 10) or drape fabricated from the fabric 12 will test at not more than 1 gram of penetration within a critical zone 11 of the respective gown 10 or drape when tested pursuant to the AATCC 42 impact penetration standardized test method. In other words, in an embodiment, a finished garment (*e.g.,* gown 10) or drape fabricated from the fabric 12 will conform to at least minimum standards established for Level 2 classification by the AAMI PB70 Standard. Additionally, in an embodiment a finished garment (*e.g.,* gown 10) or drape fabricated from the fabric 12 will maintain the above described hydrostatic resistance and impact penetration performance after at least ten institutional laundering/autoclave cycles and reliably for the full anticipated institutional service life prescribed by the manufacturer, which may significantly exceed ten cycles.

Advantageously, the coated, two-ply barrier fabric 12 described herein avoids problems of other barrier fabrics known in the art. For example, the two-ply construction of the fabric 12, where the coated side of the first ply 44 and coated side of the second ply 46 are interior 48 facing, enhances the durability of the fabric 12. By protecting the coating 42 from environmental factors (*e.g.,* institutional laundering/autoclave cycles) the useful life of the fabric 12 is extended. Other known barrier fabrics often utilize chemical hydrophobic finishes, containing fluorocarbons, that are known to diminish through repeated processing and use cycles. Leaving the coating exposed can drastically reduce the lifespan of the barrier fabric. Furthermore, the construction of the fabric 12 offers a more reliable barrier without sacrificing the feel of the fabric 12. By utilizing two plies 44, 46 with thin coatings 42, the fabric 12 can achieve the barrier properties of a fabric with a much thicker coating without compromising on the feel of the fabric. Whereas fabrics known in the art with thicker coatings typically have an uncomfortable, undesirable rubber-like feel, the fabric 12 described above maintains the feel and comfort of a woven or knitted fabric because the coated sides of the plies 44, 46 are not exposed to a user or wearer. Moreover, the two coated plies 44, 46 of the fabric 12 offer a barrier redundancy not offered by coated barrier fabrics known in the art. The two-ply configuration of the fabric 12 yields a higher hydrostatic resistance than either of the two plies 44, 46 would yield individually or would be yielded by a single layer or ply that was coated with the same quantity of coating 42 utilized cumulatively for the two individual plies 44, 46. These, other features, and combinations thereof (as described in the preceding paragraphs) improve upon the shortcomings of reusable barrier fabrics known in the art. Other advantages and technical effects of the embodiments of this invention will become evident to one skilled in the art from the preceding description.

While the present invention has been illustrated by the description of various embodiments and while these embodiments have been described in some detail, it is not the intention of the Applicant to restrict or in any way limit the scope of the invention to such detail. Additional advantages and modifications will readily appear to those skilled in the art. The invention in its broader aspects is therefore not limited to the specific details and illustrative examples shown and described. Accordingly, departures may be made from such details without departing from the scope of the claims.

## Claims

1. A barrier fabric comprising:
a first ply and a second ply each comprising a coated side and a non-coated side,
wherein the first ply and the second ply are joined together about a periphery of the first ply and the second ply to form the barrier fabric and the coated side of the first ply and
the coated side of the second ply comprise a non-fluorine containing polymer coating of silicone,
wherein the coated side of the first ply and the coated side of the second ply face each other and define an interior of the barrier fabric, with each coated side able to come into direct contact with one another but remain movable thereagainst in a transverse direction and able to form a gap therebetween,
wherein the non-coated side of the first ply and the non-coated side of the second ply face opposing directions and define an exterior of the barrier fabric,
wherein the non-fluorine containing polymer coating is applied at a rate of from 15 g/m2 to 50 g/m2, and
wherein the barrier fabric acts as a barrier against bodily fluids.

2. The fabric of claim 1, wherein the first ply and the second ply are woven or knitted.

3. The fabric of claim 1 to 5, wherein the content of the first and second ply comprise 100% polyester or 100% polyamide.

4. The fabric of at least one of claims 1 to 3, wherein the non-fluorine containing polymer coating is applied at a rate of 25 g/m2.

5. The fabric of at least one of claims 1 to 4, wherein the combined coating of the coated side of the first ply and the coated side of the second ply is applied at a rate of no more than 100 g/m2.

6. The fabric of at least one of claims 1 or 2, wherein the content of the first and second ply comprise at least 25% polyester or at least 25% polyamide.

7. The fabric of at least one of claims 1 to 6, wherein the first ply and the second ply include monofilament or multifilament yarn.

8. The fabric of at least one of claims 1 to 7, wherein the first ply and the second ply are entirely woven or knitted from monofilament or multifilament yarn.

9. A reusable medical product comprising the barrier fabric of at least one of claims 1 to 8.

10. The reusable medical product of claim 9 wherein the medical product tests for 20 cm or more of hydrostatic resistance within a critical zone of the medical product that is fabricated from the barrier fabric when tested pursuant to an AATCC 127 standardized test method.

11. The reusable medical product of claim 9, wherein the medical product tests for 1 gram or less of penetration within a critical zone of the medical product that is fabricated from the barrier fabric when tested pursuant to an AATCC 42 standardized test method.

12. The reusable medical product of claim 9, wherein the medical product tests for 20 cm or more of hydrostatic resistance within a critical zone of the medical product that is fabricated from the barrier fabric when tested pursuant to an AATCC 127 standardized test method and the medical product tests for 1 gram or less of penetration within a critical zone of the medical product that is fabricated from the barrier fabric after at least 10 institutional laundering/autoclave cycles, wherein each of the institutional laundering/autoclave cycles includes the following steps:
soaking the medical product in water at 90°-100° F for two to five minutes; thereafter, soaking the medical product in an alkali bath at 120°-160° F for three to ten minutes;
thereafter, placing the medical product in a detergent bath at 160° F for five to ten minutes, wherein the medical product is mechanically agitated in at least some of the baths; thereafter, rinsing the medical product at temperatures below 140° F to ambient temperature; thereafter, soaking the medical product in an acid sour bath in which the pH is adjusted to the 4.0 to 7.0 range; thereafter, extracting the medical product to remove water followed by drying the medical product in a dryer at an average temperature of 160° F for 15 to 30 minutes; and autoclaving the medical product at a temperature of 270° F for at least four minutes to sterilize the medical product.

13. The reusable medical product of at least one of claims 10 to 12, wherein the reusable medical product is a surgical or isolation gown, or a surgical drape.

14. A method for making a two-ply barrier fabric for use in a reusable medical product, the method comprising:
applying, via a single pass, a non-fluorine containing polymer coating of silicone to a side of each of two separate plies of a woven or knitted fabric, wherein the coating is applied at a rate of from 15 g/m2 to 50 g/m2;
curing, by a single cure process, the coating on the fabric; and
joining together the coated first ply and second ply about a periphery of each of the first ply and the second ply to form the barrier fabric,
wherein the coated side of the first ply and the coated side of the second ply face each other and define an interior of the barrier fabric, with each coated side able to come into direct contact with one another but remain movable thereagainst in a transverse direction and able to form a gap therebetween,
wherein the non-coated side of the first ply and the non-coated side of the second ply face opposing directions and define an exterior of the barrier fabric, and
wherein the barrier fabric acts as a barrier against bodily fluids and
wherein the barrier fabric is suitable for use within or as a reusable medical product.

15. The method of claim 14, wherein the reusable medical product is a surgical gown, an isolation gown, or a surgical drape.

16. The method of at least one of claims 14 to 15, wherein the content of the first and second ply comprise at least 25% polyester or at least 25% polyamide.

## Patentansprüche

1. Barrierestoff, aufweisend:
eine erste Lage und eine zweite Lage, die jeweils eine beschichtete Seite und eine unbeschichtete Seite aufweisen,
wobei die erste Lage und die zweite Lage an einem Umfang der ersten Lage und der zweiten Lage miteinander verbunden sind, um den Barrierestoff zu bilden, und wobei die beschichtete Seite der ersten Lage und die beschichtete Seite der zweiten Lage eine nicht fluorhaltige Polymerbeschichtung aus Silikon aufweisen,
wobei die beschichtete Seite der ersten Lage und die beschichtete Seite der zweiten Lage einander zugewandt sind und einen Innenraum des Barrierestoffs definieren, wobei jede der beschichteten Seiten in direkten Kontakt mit der jeweils anderen Seite kommen kann, aber in einer Querrichtung gegenüber einer solchen beweglich bleibt und zwischen diesen Schichten einen Spalt bilden kann,
wobei die unbeschichtete Seite der ersten Lage und die unbeschichtete Seite der zweiten Lage in entgegengesetzte Richtungen zeigen und eine Außenseite des Barrierestoffs definieren,
wobei die nicht fluorhaltige Polymerbeschichtung in einer Menge von zwischen 15 g/m2 und 50 g/m2 aufgebracht ist, und
wobei der Barrierestoff als Barriere gegen Körperflüssigkeiten wirkt.

2. Stoff nach Anspruch 1, wobei die erste Lage und die zweite Lage gewebt oder gestrickt sind.

3. Stoff nach wenigstens einem der Ansprüche 1 oder 2, wobei der Gehalt der ersten und zweiten Lage wenigstens 25 % Polyester oder wenigstens 25 % Polyamid aufweist.

4. Stoff nach wenigstens einem der Ansprüche 1 bis 3, wobei die nicht fluorhaltige Polymerbeschichtung in einer Menge von 25 g/m2 aufgebracht ist.

5. Stoff nach wenigstens einem der Ansprüche 1 bis 4, wobei, in Kombination, die Beschichtung der beschichteten Seite der ersten Lage und der beschichteten Seite der zweiten Lage in einer Menge von nicht mehr als 100 g/m2 aufgebracht ist.

6. Stoff nach Anspruch 1 bis 5, wobei der Gehalt der ersten und zweiten Lage 100 % Polyester oder 100 % Polyamid aufweist.

7. Stoff nach wenigstens einem der Ansprüche 1 bis 6, wobei die erste Lage und die zweite Lage Monofilament- oder Multifilamentgarn enthalten.

8. Stoff nach wenigstens einem der Ansprüche 1 bis 7, wobei die erste Lage und die zweite Lage vollständig aus Monofilament- oder Multifilamentgarn gewebt oder gestrickt sind.

9. Wiederverwendbares medizinisches Produkt, das den Barrierestoff nach wenigstens einem der Ansprüche 1 bis 8 aufweist.

10. Wiederverwendbares medizinisches Produkt nach Anspruch 9, wobei das medizinische Produkt bei einer Prüfung gemäß einer standardisierten Prüfmethode nach AATCC 127 eine hydrostatische Beständigkeit von 20 cm oder mehr innerhalb einer kritischen Zone des medizinischen Produkts, die aus dem Barrierestoff hergestellt ist, aufweist.

11. Wiederverwendbares medizinisches Produkt nach Anspruch 9, wobei das medizinische Produkt bei einer Prüfung gemäß einer standardisierten Prüfmethode nach AATCC 42 eine Penetration von 1 Gramm oder weniger innerhalb einer kritischen Zone des medizinischen Produkts, die aus dem Barrierestoff hergestellt ist, aufweist.

12. Wiederverwendbares medizinisches Produkt nach Anspruch 9, wobei das medizinische Produkt bei einer Prüfung gemäß einer standardisierten Prüfmethode nach AATCC 127 eine hydrostatische Beständigkeit von 20 cm oder mehr innerhalb einer kritischen Zone des medizinischen Produkts, die aus dem Barrierestoff hergestellt ist, aufweist und das medizinische Produkt nach wenigstens 10 institutionellen Waschzyklen / Autoklavierzyklen eine Penetration von 1 Gramm oder weniger innerhalb einer kritischen Zone des medizinischen Produkts, die aus dem Barrierestoff hergestellt ist, aufweist, wobei jeder der institutionellen Wasch- / Autoklavierzyklen die folgenden Schritte aufweist:
das medizinische Produkt wird für zwei bis fünf Minuten in Wasser mit einer Temperatur von 90 °F bis 100 °F eingetaucht; anschließend wird das medizinische Produkt für drei bis zehn Minuten in ein alkalisches Bad mit einer Temperatur von 120 °F bis 160 °F eingetaucht; anschließend wird das medizinische Produkt für fünf bis zehn Minuten in ein Waschmittelbad mit einer Temperatur von 160 °F gelegt, wobei das medizinische Produkt in wenigstens manchen der Bäder mechanisch bewegt wird; anschließend wird das medizinische Produkt bei Temperaturen unter 140 °F bis zur Umgebungstemperatur gespült; anschließend wird das medizinische Produkt in ein saures Bad eingetaucht, in dem der pH-Wert auf einen Bereich von 4,0 bis 7,0 eingestellt ist; anschließend wird das medizinische Produkt herausgenommen, um Wasser zu entfernen, gefolgt von einer Trocknung des medizinischen Produkts für 15 bis 30 Minuten in einem Trockner bei einer durchschnittlichen Temperatur von 160 °F; und dann wird das medizinische Produkt für wenigstens vier Minuten bei einer Temperatur von 270 °F autoklaviert, um das medizinische Produkt zu sterilisieren.

13. Wiederverwendbares medizinisches Produkt nach wenigstens einem der Ansprüche 10 bis 12, wobei das wiederverwendbare medizinische Produkt ein Operations- oder Isolationskittel oder ein OP-Tuch ist.

14. Verfahren zur Herstellung eines zweilagigen Barrierestoffs zur Verwendung in einem wiederverwendbaren medizinischen Produkt, wobei das Verfahren Folgendes aufweist:
Auftragen einer nicht fluorhaltigen Polymerbeschichtung aus Silikon in einem einzigen Durchgang auf eine Seite einer jeden von zwei separaten Lagen eines gewebten oder gestrickten Stoffs, wobei die Beschichtung in einer Menge von zwischen 15 g/m2 und 50 g/m2 aufgetragen wird;
Aushärten der Beschichtung auf dem Stoff in einem einzigen Aushärtungsprozess; und
Verbinden der beschichteten ersten Lage und der beschichteten zweiten Lage an einem Umfang einer jeden der ersten Lage und der zweiten Lage, um den Barrierestoff zu bilden,
wobei die beschichtete Seite der ersten Lage und die beschichtete Seite der zweiten Lage einander zugewandt sind und einen Innenraum des Barrierestoffs definieren, wobei jede der beschichteten Seiten in direkten Kontakt mit der jeweils anderen Seite kommen kann, aber in einer Querrichtung gegenüber einer solchen beweglich bleibt und zwischen diesen Schichten einen Spalt bilden kann,
wobei die unbeschichtete Seite der ersten Lage und die unbeschichtete Seite der zweiten Lage in entgegengesetzte Richtungen zeigen und eine Außenseite des Barrierestoffs definieren, und
wobei der Barrierestoff als Barriere gegen Körperflüssigkeiten wirkt und
wobei der Barrierestoff zur Verwendung in einem wiederverwendbaren medizinischen Produkt oder als wiederverwendbares medizinisches Produkt geeignet ist.

15. Verfahren nach Anspruch 14, wobei das wiederverwendbare medizinische Produkt ein Operationskittel, ein Isolationskittel oder ein OP-Tuch ist.

16. Verfahren nach wenigstens einem der Ansprüche 14 bis 15, wobei der Gehalt der ersten und zweiten Lage wenigstens 25 % Polyester oder wenigstens 25 % Polyamid aufweist.

## Revendications

1. Tissu barrière comprenant :
une première couche et une deuxième couche comprenant chacune un côté revêtu et un côté non revêtu,
dans lequel la première couche et la deuxième couche sont assemblées autour d'une périphérie de la première et de la deuxième couche pour former le tissu barrière et le côté revêtu de la première couche ainsi que le côté revêtu de la deuxième couche comprennent un revêtement polymère de silicone ne contenant pas de fluor,
dans lequel le côté revêtu de la première couche et le côté revêtu de la deuxième couche se font face et définissent un intérieur du tissu barrière, chaque côté revêtu étant apte à entrer en contact direct l'un avec l'autre mais restant mobile contre celui-ci dans une direction transversale et étant apte à former un espace entre eux,
dans lequel le côté non revêtu de la première couche et le côté non revêtu de la deuxième couche sont orientés dans des directions opposées et définissent un extérieur du tissu barrière,
dans lequel le revêtement polymère ne contenant pas de fluor est appliqué à un taux allant de 15 g/m2 à 50 g/m2, et
dans lequel le tissu barrière sert de barrière aux fluides corporels.

2. Tissu selon la revendication 1, dans lequel la première couche et la deuxième couche sont tissées ou tricotées.

3. Tissu selon au moins l'une des revendications 1 ou 2, dans lequel le contenu des première et deuxième couches comprend au moins 25 % de polyester ou au moins 25 % de polyamide.

4. Tissu selon au moins l'une des revendications 1 à 3, dans lequel le revêtement polymère ne contenant pas de fluor est appliqué à un taux de 25 g/m2.

5. Tissu selon au moins l'une des revendications 1 à 4, dans lequel le revêtement combiné du côté revêtu de la première couche et du côté revêtu de la deuxième couche est appliqué à un taux ne dépassant pas 100 g/m2.

6. Tissu selon la revendication 1 à 5, dans lequel le contenu des première et deuxième couches comprend 100 % de polyester ou 100 % de polyamide.

7. Tissu selon au moins l'une des revendications 1 à 6, dans lequel la première couche et la deuxième couche comportent un fil monofilament ou multifilament.

8. Tissu selon au moins l'une des revendications 1 à 7, dans lequel la première couche et la deuxième couche sont entièrement tissées ou tricotées à partir de fil monofilament ou multifilament.

9. Produit médical réutilisable comprenant le tissu barrière selon au moins l'une des revendications 1 à 8.

10. Produit médical réutilisable selon la revendication 9 dans lequel le produit médical présente une résistance hydrostatique de 20 cm ou plus dans une zone critique du produit médical qui est fabriqué à partir du tissu barrière lorsqu'il est testé conformément à une méthode de test normalisée AATCC 127.

11. Produit médical réutilisable selon la revendication 9, dans lequel le produit médical présente une pénétration de 1 gramme ou moins dans une zone critique du produit médical qui est fabriqué à partir du tissu barrière lorsqu'il est testé conformément à une méthode de test normalisée AATCC 42.

12. Produit médical réutilisable selon la revendication 9, dans lequel le produit médical présente une résistance hydrostatique de 20 cm ou plus dans une zone critique du produit médical qui est fabriqué à partir du tissu barrière lorsqu'il est testé conformément à une méthode de test normalisée AATCC 127 et le produit médical présente une pénétration de 1 gramme ou moins dans une zone critique du produit médical qui est fabriqué à partir du tissu barrière après au moins 10 cycles de lavage/autoclave institutionnels, dans lequel chaque cycle de lavage/autoclave comporte les étapes suivantes :
trempage du produit médical dans de l'eau à 90 °-100 °F pendant deux à cinq minutes ; ensuite, trempage du produit médical dans un bain alcalin à 120 °-160 °F pendant trois à dix minutes ; ensuite, placement du produit médical dans un bain détergent à 160 °F pendant cinq à dix minutes, dans lequel le produit médical est agité mécaniquement dans au moins certains des bains ; ensuite, rinçage du produit médical à des températures inférieures à 140 °F jusqu'à atteindre la température ambiante ; ensuite, trempage du produit médical dans un bain acide dans lequel le pH est ajusté à la plage de 4,0 à 7,0 ; ensuite, extraction du produit médical pour éliminer de l'eau, puis séchage du produit médical dans un séchoir à une température moyenne de 160 °F pendant 15 à 30 minutes ; et
autoclavage du produit médical à une température de 270 °F pendant au moins quatre minutes pour stériliser le produit médical.

13. Produit médical réutilisable selon au moins l'une des revendications 10 à 12, dans lequel le produit médical réutilisable est une blouse chirurgicale ou d'isolation, ou un drap chirurgical.

14. Procédé pour fabriquer un tissu barrière à deux couches pour une utilisation dans un produit médical réutilisable, le procédé comprenant :
l'application, par un seul passage, d'un revêtement polymère de silicone ne contenant pas de fluor sur un côté de chacune de deux couches distinctes d'un tissu ou tricot, dans lequel le revêtement est appliqué à un taux allant de 15 g/m2 à 50 g/m2 ;
le durcissement, par un seul processus de durcissement, du revêtement sur le tissu ; et
l'assemblage de la première couche et de la deuxième couche revêtues autour d'une périphérie de chacune de la première couche et de la deuxième couche pour former le tissu barrière,
dans lequel le côté revêtu de la première couche et le côté revêtu de la deuxième couche se font face et définissent un intérieur du tissu barrière, chaque côté revêtu étant apte à entrer en contact direct l'un avec l'autre mais restant mobile contre celui-ci dans une direction transversale et étant apte à former un espace entre eux,
dans lequel le côté non revêtu de la première couche et le côté non revêtu de la deuxième couche sont orientés dans des directions opposées et définissent un extérieur du tissu barrière, et
dans lequel le tissu barrière sert de barrière aux fluides corporels et
dans lequel le tissu barrière est adapté à une utilisation interne ou comme produit médical réutilisable.

15. Procédé selon la revendication 14, dans lequel le produit médical réutilisable est une blouse chirurgicale, une blouse d'isolation ou un drap chirurgical.

16. Procédé selon au moins l'une des revendications 14 à 15, dans lequel le contenu des première et deuxième couches comprend au moins 25 % de polyester ou au moins 25 % de polyamide.
